(19) Europäisches Patentamt
European Patent Office
Office européen des brevets

(11) EP 0 659 396 B1

(12) **FASCICULE DE BREVET EUROPEEN**

(45) Date de publication et mention
de la délivrance du brevet:
**07.04.1999 Bulletin 1999/14**

(51) Int. Cl.$^6$: **A61K 7/13**, A45D 19/00

(21) Numéro de dépôt: **94402436.3**

(22) Date de dépôt: **27.10.1994**

(54) **Procédé de coloration d'oxydation des fibres kératiniques humaines à l'aide de vapeur d'eau**

Verfahren zum oxydatieven Färben von keratinen Fasern unter Anwendung von Wasserdampf

Process for oxidative hair dyeing using steam

(84) Etats contractants désignés:
**AT BE CH DE ES FR GB IT LI NL SE**

(30) Priorité: **22.12.1993 FR 9315481**

(43) Date de publication de la demande:
**28.06.1995 Bulletin 1995/26**

(73) Titulaire: **L'OREAL**
**75008 Paris (FR)**

(72) Inventeurs:
• **Samain, Henri**
  **F-91570 Bievres (FR)**

• **Sturla, Jean-Michel**
  **F-92210 Saint-Cloud (FR)**

(74) Mandataire: **Goulard, Sophie et al**
**L'OREAL,**
**Département Propriété Industrielle,**
**90, rue du Général Roguet**
**92583 Clichy Cedex (FR)**

(56) Documents cités:
**CH-A- 357 161        FR-A- 1 011 151**
**FR-A- 2 273 492**

## Description

La présente invention est relative à un procédé de coloration (ou teinture) d'oxydation des fibres kératiniques mettant en oeuvre de la vapeur d'eau et une composition comprenant au moins un colorant d'oxydation.

Il est connu de teindre les fibres kératiniques, et en particulier les cheveux humains, avec des compositions de teinture contenant des précurseurs de colorants d'oxydation, en particulier des ortho- ou paraphénylènediamines, des ortho- ou para-aminophénols, composés généralement appelés "bases d'oxydation", et qui sont le plus souvent associés à des coupleurs encore appelés modificateurs de coloration, plus particulièrement des métaphénylènediamines, des métaaminophénols et des métadiphénols, qui permettent de modifier et d'enrichir en reflets les colorations "de fond" obtenues avec les produits de condensation des bases d'oxydation.

Or, ce type de procédé de coloration, qui nécessite par ailleurs la mise en oeuvre d'un agent oxydant tel que le peroxyde d'hydrogène, donne des résultats tinctoriaux faibles lorsque les fibres kératiniques à colorer sont très sensibilisées (i.e "abîmées").

En d'autres termes, cette coloration d'oxydation présente l'inconvénient d'être sélective au niveau de la fibre à colorer.

On appelle sélectivité d'un colorant, la différence de montée (i.e de pouvoir de coloration) de celui-ci sur la fibre capillaire selon que celle-ci a été plus ou moins sensibilisée, soit par un traitement tel qu'une décoloration ou une permanente, soit par les agents atmosphériques notamment dans le cas des pointes des cheveux.

Les résultats de coloration obtenus sur des cheveux présentant des différences de sensibilisation sont donc hétérogènes. Ces irrégularités ne sont bien évidemment pas souhaitables d'un point de vue esthétique.

La présente invention vise à résoudre le problème ci-dessus.

La demanderesse a maintenant découvert de façon surprenante que l'utilisation d'un gaz chauffé à une température supérieure à 75°C, comprenant de la vapeur d'eau, sur des cheveux traités avec une composition comprenant au moins un colorant d'oxydation, et ceci en présence d'un agent oxydant, permettait d'obtenir des résultats tinctoriaux dépendant peu ou pas du tout du degré de sensibilisation des fibres kératiniques à colorer.

Selon l'invention, les cheveux sont ainsi colorés de façon uniforme sur l'ensemble de la chevelure, des racines aux pointes, et ceci quel que soit l'état du cheveu.

On notera que l'utilisation de la vapeur d'eau dans un procédé de coloration d'oxydation a déjà été décrite dans le brevet FR-1011151, document dans lequel de la vapeur d'eau chauffée à environ 50°C est mise en oeuvre dans le but d'accélérer le processus de coloration des cheveux tout en diminuant les doses de teintures employées. Toutefois, à cette température, il n'y a pas de diminution de la sélectivité au sens défini ci-avant.

La présente invention concerne donc un procédé de teinture d'oxydation des fibres kératiniques, caractérisé par le fait qu'il consiste à mettre en contact, en présence d'un agent oxydant, des fibres kératiniques sur lesquelles on a appliqué une composition tinctoriale contenant au moins un colorant d'oxydation, avec un gaz contenant de la vapeur d'eau, la température dudit gaz étant supérieure à 75°C, le temps de contact entre ledit gaz et lesdites fibres à colorer étant inférieur à deux minutes.

En plus de la vapeur d'eau, le gaz vecteur peut contenir de la vapeur de solvant, des gaz tels que l'oxygène, l'azote, des mélange de gaz tels que l'air ou des composés vaporisables.

Les solvants utilisables pour la production de vapeur sont des solvants organiques cosmétiquement acceptables et plus particulièrement des alcools tels que l'éthanol, l'isopropanol, l'alcool benzylique, l'alcool phényléthylique ou des glycols ou éthers de glycol tels que par exemple les éthers monométhylique, monoéthylique et monobutylique de l'éthylèneglycol, le propylèneglycol, le butylèneglycol, le dipropylèneglycol ainsi que les alkyléthers comme le monobutyléther du diéthylèneglycol.

Le gaz comprend de préférence au moins 1% en volume de vapeur d'eau par rapport au volume total du gaz.

Le gaz est constitué de préférence soit uniquement ou essentiellement de vapeur d'eau, soit d'un mélange de vapeur d'eau et d'air.

La température du gaz est de préférence supérieure ou égale à 85°C et plus particulièrement comprise entre 85 et 150°C.

En particulier, le gaz est mis en contact avec la fibre à colorer pendant une durée allant de 0,01 seconde à 2 minutes.

De préférence, le gaz est mis en contact avec la fibre pendant une durée allant de 0,1 seconde à 50 secondes et encore plus préférentiellement de 1 à 10 secondes.

L'application du gaz peut être répétée plusieurs fois sur la même fibre, chaque opération se faisant selon la durée indiquée ci-dessus.

Dans un premier mode de mise en oeuvre du procédé selon l'invention qui est ici préféré, on applique sur les cheveux une composition tinctoriale contenant au moins un colorant d'oxydation, puis on les soumet à l'action de la vapeur d'eau.

Selon d'autres modes de mise en oeuvre du procédé, il est également possible d'appliquer simultanément la composition tinctoriale et le gaz comprenant de la vapeur d'eau.

Il est également possible de faire parvenir sur les cheveux tout ou partie de la composition tinctoriale à l'aide du flux de gaz lorsque certains ou tous les constituants de la composition tinctoriale sont entraînables ou vaporisables.

L'agent oxydant est, quant à lui, soit ajouté avant emploi à la composition tinctoriale, soit appliqué simultanément ou juste après la composition tinctoriale dans un support approprié. Le support approprié peut être une composition aqueuse ou le gaz contenant la vapeur d'eau lui-même.

Dans un mode particulier de réalisation de l'invention, l'application de vapeur d'eau est suivie d'un rinçage à l'eau.

La production d'un gaz chaud comprenant de la vapeur d'eau peut se faire à l'aide de tout appareil connu en soi. Toutefois, selon la présente invention, on utilise de préférence un appareil tel que celui décrit dans le brevet français FR-B-2273492, ou tout autre appareil équivalent, qui convient particulièrement bien.

Les colorants d'oxydation, bases et coupleurs, sont des produits bien connus de l'état de la technique. Des colorants de ce type sont cités par exemple dans le brevet FR-B-2575067 et dans la demande de brevet EP-A-496653, dont les enseignements sont ici totalement inclus à titre de référence.

Le (ou les) colorant(s) d'oxydation est (sont) présent(s) dans des concentrations allant de préférence de 0,001 à 10% en poids environ par rapport au poids total de la composition tinctoriale.

L'agent oxydant est choisi de préférence parmi le peroxyde d'hydrogène, le peroxyde d'urée, les bromates de métaux alcalins, les persels tels que les perborates et les persulfates. Le peroxyde d'hydrogène est particulièrement préféré.

Le pH de la composition tinctoriale est généralement compris entre 3 et 11 environ. Il peut être ajusté à la valeur désirée au moyen d'agents alcalinisants habituellement utilisés en teinture des fibres kératiniques, tels que l'ammoniaque, les carbonates alcalins, les alcanolamines telles que par exemple les mono-, di- et triéthanolamines ainsi que leurs dérivés, les hydroxydes de sodium ou de potassium, ou au moyen d'agents acidifiants classiques, tels que les acides minéraux ou organiques comme par exemple les acides chlorhydrique, tartrique, citrique et phosphorique.

Le pH de la composition renfermant l'agent oxydant est choisi de telle manière qu'après mélange avec la composition tinctoriale, le pH de la composition résultante appliquée sur les fibres kératiniques varie de préférence entre 3 et 11. Il est ajusté à la valeur désirée à l'aide d'agents acidifiants ou éventuellement alcalinisants bien connus de l'état de la technique, notamment tels que décrits ci-dessus.

La composition oxydante contient de préférence de l'eau oxygénée.

Les compositions tinctoriales peuvent également contenir, dans leur forme de réalisation préférée, des agents tensioactifs anioniques, cationiques, nonioniques, amphotères, bien connus de l'état de la technique, ou leurs mélanges, dans des proportions comprises entre 0,5 et 55 % en poids environ, et de préférence entre 2 et 50 % en poids par rapport au poids total de la composition tinctoriale.

Les compositions tinctoriales peuvent également contenir des solvants organiques pour solubiliser les composants qui ne seraient pas suffisamment solubles dans l'eau. Parmi eux, on peut citer à titre d'exemple, les alcanols inférieurs en $C_1$-$C_4$, tels que l'éthanol et l'isopropanol ; le glycérol ; les glycols ou éthers de glycols comme le 2-butoxyéthanol, le propylène glycol, le monoéthyléther et le monométhyléther du diéthylèneglycol ; les alcools aromatiques comme l'alcool benzylique ou le phénoxyéthanol, les produits analogues et leurs mélanges.

Ces solvants sont présents de préférence dans des proportions comprises entre environ 1 et 40 % en poids, et en particulier entre 5 et 30 % en poids par rapport au poids total de la composition tinctoriale.

Les compositions tinctoriales peuvent en outre contenir des agents épaississants organiques tels que les polymères d'acide acrylique éventuellement réticulés, ou des agents épaississants minéraux tels que la bentonite, présents de préférence dans des proportions comprises entre environ 0,1 et 5 %, et en particulier entre 0,2 et 3 % en poids par rapport au poids total de la composition tinctoriale.

Des agents antioxydants peuvent également être introduits dans les compositions tinctoriales. Ils sont choisis en particulier parmi le sulfite de sodium, l'acide thioglycolique, l'acide thiolactique, le bisulfite de sodium, l'acide déhydroascorbique, l'hydroquinone, la 2-méthyl hydroquinone, la 2-tertiobutyl hydroquinone et l'acide homogentisique et sont en général présents dans des proportions comprises entre environ 0,05 et 1,5 % en poids par rapport au poids total de la composition tinctoriale.

Les compositions tinctoriale peuvent également contenir d'autres adjuvants cosmétiquement acceptables, tels que par exemple des agents de pénétration, des agents séquestrants, des parfums, des tampons, des agents dispersants, des agents de traitement, des agents de conditionnement, des agents filmogènes, des agents conservateurs, des agents opacifiants.

La composition tinctoriale utilisée dans le procédé selon l'invention peut se présenter sous des formes habituellement utilisées pour la teinture des cheveux telle que de liquide plus ou moins épaissi ou gélifié, de crème, de mousse en aérosol ou sous toute autre forme appropriée pour réaliser une teinture des cheveux.

Les exemples qui suivent illustrent l'invention.

EP 0 659 396 B1

## EXEMPLES

### EXEMPLE 1 (invention)

On prépare la composition tinctoriale suivante :

| | |
|---|---|
| - Résorcine | 0,01 g |
| - Paraphénylènediamine | 0,4 g |
| - Paraaminophénol | 0,24 g |
| - 1-méthyl 2-hydroxy 4-(2'-hydroxyéthyl)aminobenzène | 1,2 g |
| - Nonylphénol polyoxyéthyléné à 9 moles d'oxyde d'éthylène | 3 g |
| - Alcool oléique | 18 g |
| - Polymère constitué de motifs récurrents de formule : | |

$$\left[ \begin{array}{c} CH_3 \\ | \\ -N^+- \\ | \\ CH_3 \end{array} \ Cl^- \ (CH_2)_3 - \begin{array}{c} CH_3 \\ | \\ N^+- \\ | \\ CH_3 \end{array} \ Cl^- \ (CH_2)_6 \right]-$$

| | |
|---|---|
| préparé selon le brevet FR-2270846 | 3 g |
| - Alcool éthylique | 9 g |
| - Alcool benzylique | 11 g |
| - Acide éthylène diamine tétraacétique | 0,2 g |
| - Ammoniaque (solution aqueuse à 22% de $NH_3$) | 12,9 g |
| - Monoéthanolamine | 6,5 g |
| - Thiolactate d'ammonium (50% MA en acide thiolactique) | 0,8 g |
| - 1-phényl 3-méthyl 5-pyrazolone | 0,15 g |
| - Eau déminéralisée q.s.p. | 100 g |

Cette composition est mélangée poids pour poids avec de l'eau oxygénée à 20 volumes au moment de l'emploi.

On applique le mélange obtenu ci-dessus sur une mèche de cheveux décolorés (solubilité alcaline SA20), (mèche n°1) et sur une mèche de ces mêmes cheveux ayant subis deux permanentes (mèche n°2).

On envoie ensuite sur les deux mèches deux jets de vapeur d'eau à 90°C pendant 30 secondes chacun. Les mèches sont lavées avec un shampooing standard puis séchées.

Les coordonnées chromatiques dans le système L, a, b (indicatives des résultats de coloration) sont mesurées sur chacune des deux mèches avec un colorimètre MINOLTA CHROMA METER CR 200.
L'écart de luminance ($\Delta L$) entre la mèche n°2 et la mèche n°1 est le suivant :

$$\Delta L = L_2 - L_1 = -1,7$$

On observe donc que les nuances sont à peu près identiques.

4

### EXEMPLE 2 (comparatif)

On applique le mélange de l'exemple 1 sur une mèche de cheveux décolorés (solubilité alcaline SA20), (mèche n°1) et sur une mèche de ces mêmes cheveux ayant subis deux permanentes (mèche n°2). On laisse pauser la composition pendant 30 minutes à température ambiante. Les mèches sont lavées avec un shampooing standard puis séchées.

Les coordonnées chromatiques dans le système L, a, b (indicatives des résultats de coloration) sont mesurées sur chacune des deux mèches avec un colorimètre MINOLTA CHROMA METER CR 200.

L'écart de luminance ($\Delta$L) entre la mèche n°2 et la mèche n°1 est le suivant :

$$\Delta L = L_2 - L_1 = 9,8$$

La mèche n°2 est beaucoup moins foncée que la mèche n°1. La différence de coloration entre les 2 mèches est très importante avec ce procédé non conforme à l'invention.

### EXEMPLE 3 (comparatif)

On applique le mélange de l'exemple 1 sur une mèche de cheveux décolorés (solubilité alcaline SA20), (mèche n°1) et sur une mèche de ces mêmes cheveux ayant subis deux permanentes (mèche n°2). On envoie ensuite sur les deux mèches un jet de vapeur d'eau à 50°C pendant une minute. Les mèches sont lavées avec un shampooing standard puis séchées.

Les coordonnées chromatiques dans le système L, a, b (indicatives des résultats de coloration) sont mesurées sur chacune des deux mèches avec un colorimètre MINOLTA CHROMA METER CR 200.

L'écart de luminance ($\Delta$L) entre la mèche n°2 et la mèche n°1 est le suivant :

$$\Delta L = L_2 - L_1 = 9$$

La mèche n°2 est beaucoup moins foncée que la mèche n°1. La différence de coloration entre les 2 mèches est très importante avec ce procédé non conforme à l'invention.

### EXEMPLE 4 (comparatif)

On procède de la même façon qu'à l'exemple 3, à cette différence près que l'on applique la vapeur d'eau à 50°C pendant 15 minutes.

Les coordonnées chromatiques dans le système L, a, b (indicatives des résultats de coloration) sont mesurées sur chacune des deux mèches avec un colorimètre MINOLTA CHROMA METER CR 200.

L'écart de luminance ($\Delta$L) entre la mèche n°2 et la mèche n°1 est le suivant :

$$\Delta L = L_2 - L_1 = 5$$

La mèche n°2 est beaucoup moins foncée que la mèche n°1. La différence de coloration entre les 2 mèches est très importante avec ce procédé non conforme à l'invention.

### EXEMPLE 5 (invention)

On prépare la composition tinctoriale suivante :

| | |
|---|---|
| - Paraphénylènediamine | 0,65 g |
| - Paraaminophénol | 0,03 g |
| - Résorcine | 0,25 g |
| - 1-méthyl 2-hydroxy 4-(2'-hydroxyéthyl)amino benzène | 0,02 g |
| - Nonylphénol polyoxyéthyléné à 9 moles d'oxyde d'éthylène | 3 g |
| - Alcool oléique | 18 g |

- Polymère constitué de motifs récurrents de formule :

$$\left[ \begin{array}{c} CH_3 \\ | \\ N^+ \\ | \\ CH_3 \end{array} \overset{Cl^-}{\underset{(CH_2)_3}{\quad}} \begin{array}{c} CH_3 \\ | \\ N^+ \\ | \\ CH_3 \end{array} \overset{Cl^-}{\underset{(CH_2)_6}{\quad}} \right]$$

| | |
|---|---|
| préparé selon le brevet FR-2270846 | 3 g |
| - Alcool éthylique | 9 g |
| - Alcool benzylique | 11 g |
| - Acide éthylène diamine tétraacétique | 0,2 g |
| - Ammoniaque (solution aqueuse à 22% de $NH_3$) | 12,9 g |
| - Monoéthanolamine | 6,5 g |
| - Thiolactate d'ammonium (50% MA en acide thiolactique) | 0,8 g |
| - 1-phényl 3-méthyl 5-pyrazolone | 0,15 g |
| - Eau déminéralisée q.s.p. | 100 g |

Cette composition est mélangée poids pour poids avec de l'eau oxygénée à 20 volumes au moment de l'emploi.

On applique le mélange obtenu ci-dessus sur des cheveux gris naturels à 90 % de blancs et sur les mêmes cheveux permanentés deux fois, puis on envoie un jet de vapeur d'eau à 90°C pendant 15 à 20 secondes. Les cheveux sont ensuite lavés avec un shampooing standard et séchés.

Les cheveux sont teints en châtain et la différence de coloration (sélectivité) est faible entre les deux types de cheveux.

### EXEMPLE 6 (invention)

On utilise une composition tinctoriale similaire à celle de l'exemple 5, à cette différence que l'on modifie les quantités d'ammoniaque (solution aqueuse à 22% de $NH_3$) et de monoéthanolamine

| | |
|---|---|
| Ammoniaque (solution aqueuse à 22% de $NH_3$) | 0,56 g |
| Monoéthanolamine | 12 g |

Cette composition est mélangée poids pour poids avec de l'eau oxygénée à 20 volumes au moment de l'emploi.

On applique le mélange obtenu ci-dessus sur des cheveux gris naturels à 90 % de blancs et sur les mêmes cheveux permanentés deux fois, puis on envoie un jet de vapeur d'eau à 90°C pendant 30 secondes. Les cheveux sont ensuite lavés avec un shampooing standard et séchés.

Les cheveux sont teints en châtain et la sélectivité est faible entre les deux types de cheveux.

**EXEMPLE 7 (invention)**

On prépare, au moment de l'emploi, la composition tinctoriale suivante :

| | |
|---|---|
| - Paraphénylènediamine | 1,75 g |
| - Paraaminophénol | 0,023 g |
| - 1,3-dihydroxybenzène | 0,417 g |
| - Métaaminophénol | 0,013 g |
| - Alcool cétylique et stéarylique (50/50 en poids) | 18 g |
| - 2-octyl dodécanol | 3 g |
| - Alcool cétylstéarylique oxyéthyléné à 15 moles d'oxyde d'éthylène | 3 g |
| - Laurylsulfate d'ammonium (30% MA en solution aqueuse) | 12 g |
| - Monoéthanolamine | 12 g |
| - Eau oxygénée à 20 volumes | 40 g |
| - Acide citrique q.s. | pH 6 |
| - Eau déminéralisée q.s.p. | 100 g |

On applique cette composition sur des cheveux gris naturels à 90 % de blancs et sur les mêmes cheveux permanentés deux fois, puis on envoie un jet de vapeur d'eau à 90°C pendant 3 secondes et on laisse refroidir pendant 10 secondes. Les cheveux sont ensuite lavés avec un shampooing standard et séchés.
Les cheveux sont teints en châtain foncé et la sélectivité est faible entre les deux types de cheveux.

**Revendications**

1.   Procédé de teinture d'oxydation des fibres kératiniques, caractérisé par le fait qu'il consiste à mettre en contact, en présence d'un agent oxydant, des fibres kératiniques sur lesquelles on a appliqué une composition tinctoriale contenant au moins un colorant d'oxydation, avec un gaz contenant de la vapeur d'eau, la température dudit gaz étant supérieure à 75°C, le temps de contact entre ledit gaz et lesdites fibres à colorer étant inférieur à deux minutes.

2.   Procédé selon la revendication 1, caractérisé par le fait que le gaz a une température supérieure ou égale à 85°C.

3.   Procédé selon la revendication 2, caractérisé par le fait que le gaz a une température comprise entre 85 et 150°C.

4.   Procédé selon l'une quelconque des revendications précédentes, caractérisé par le fait que le gaz est mis en contact avec la fibre à colorer pendant une durée allant de 0,01 seconde à 2 minutes.

5.   Procédé selon la revendication 4, caractérisé par le fait que le gaz est mis en contact avec la fibre à colorer pendant une durée allant de 0,1 seconde à 50 secondes.

6.   Procédé selon la revendication 5, caractérisé par le fait que le gaz est mis en contact avec la fibre à colorer pendant une durée allant de 1 seconde à 10 secondes.

7.   Procédé selon l'une quelconque des revendications précédentes, caractérisé par le fait que l'application du gaz est répétée plusieurs fois sur une même fibre.

8. Procédé selon l'une quelconque des revendications précédentes, caractérisé par le fait que le gaz contient uniquement de la vapeur d'eau.

9. Procédé selon l'une quelconque des revendications 1 à 7, caractérisé par le fait que le gaz contient de la vapeur d'eau et au moins un autre composé sous forme de gaz ou de vapeur.

10. Procédé selon la revendication 9, caractérisé par le fait que le gaz contient de la vapeur d'eau et de l'air.

11. Procédé selon l'une quelconque des revendications précédentes, caractérisé par le fait que les colorants d'oxydation sont choisis parmi les bases d'oxydation et les mélanges de bases d'oxydation et de coupleurs.

12. Procédé selon l'une quelconque des revendications précédentes, caractérisé par le fait que l'agent oxydant est choisi parmi le peroxyde d'hydrogène, le peroxyde d'urée, les bromates de métaux alcalins et les persels.

## Claims

1. Process for the oxidation dyeing of keratinous fibres, characterized in that it consists in bringing into contact, in the presence of an oxidizing agent, keratinous fibres to which has been applied a dye composition containing at least one oxidation dye, with a gas containing water vapour, the temperature of the said gas being greater than 75°C and the contact time between the said gas and the said fibres to be dyed being less than two minutes.

2. Process according to Claim 1, characterized in that the gas is at a temperature higher than or equal to 85°C.

3. Process according to Claim 2, characterized in that the gas is at a temperature between 85 and 150°C.

4. Process according to any one of the preceding claims, characterized in that the gas is brought into contact with the fibre to be dyed for a period ranging from 0.01 second to 2 minutes.

5. Process according to Claim 4, characterized in that the gas is brought into contact with the fibre to be dyed for a period ranging from 0.1 second to 50 seconds.

6. Process according to Claim 5, characterized in that the gas is brought into contact with the fibre to be dyed for a period ranging from 1 second to 10 seconds.

7. Process according to any one of the preceding claims, characterized in that the application of the gas is repeated several times on the same fibre.

8. Process according to any one of the preceding claims, characterized in that the gas exclusively contains water vapour.

9. Process according to any one of Claims 1 to 7, characterized in that the gas contains water vapour and at least one other compound in gas or vapour form.

10. Process according to Claim 9, characterized in that the gas contains water vapour and air.

11. Process according to any one of the preceding claims, characterized in that the oxidation dyes are chosen from oxidation bases and mixtures of oxidation bases and couplers.

12. Process according to any one of the preceding claims, characterized in that the oxidizing agent is chosen from hydrogen peroxide, urea peroxide, alkali metal bromates and persalts.

## Patentansprüche

1. Verfahren zum oxidativen Färben von Keratinfasern, dadurch gekennzeichnet, daß es darin besteht, Keratinfasern, auf die eine Färbemittelzusammensetzung aufgetragen wurde, die mindestens ein Oxidationsfärbemittel enthält, in Gegenwart eines Oxidationsmittels mit einem Gas, das Wasserdampf enthält, in Kontakt zu bringen, wobei die Temperatur des Gases über 75 °C liegt und die Kontaktzeit zwischen dem Gas und den zu färbenden Fasern weniger als 2 min beträgt.

2. Verfahren nach Anspruch 1, dadurch gekennzeichnet, daß das Gas eine Temperatur von mindestens 85 °C aufweist.

3. Verfahren nach Anspruch 2, dadurch gekennzeichnet, daß das Gas eine Temperatur im Bereich von 85 bis 150 °C aufweist.

4. Verfahren nach einem der vorhergehenden Ansprüche, dadurch gekennzeichnet, daß das Gas mit der zu färbenden Faser während einer Zeitdauer von 0,01 s bis 2 min in Kontakt gebracht wird.

5. Verfahren nach Anspruch 4, dadurch gekennzeichnet, daß das Gas mit der zu färbenden Faser für eine Zeitdauer von 0,1 bis 50 s in Kontakt gebracht wird.

6. Verfahren nach Anspruch 5, dadurch gekennzeichnet, daß das Gas mit der zu färbenden Faser für eine Zeitdauer von 1 bis 10 s in Kontakt gebracht wird.

7. Verfahren nach einem der vorhergehenden Ansprüche, dadurch gekennzeichnet, daß die Anwendung des Gases mehrmals an der gleichen Faser wiederholt wird.

8. Verfahren nach einem der vorhergehenden Ansprüche, dadurch gekennzeichnet, daß das Gas nur Wasserdampf enthält.

9. Verfahren nach einem der Ansprüche 1 bis 7, dadurch gekennzeichnet, daß das Gas Wasserdampf und mindestens eine weitere Verbindung in Form von Gas oder Dampf enthält.

10. Verfahren nach Anspruch 9, dadurch gekennzeichnet, daß das Gas Wasserdampf und Luft enthält.

11. Verfahren nach einem der vorhergehenden Ansprüche, dadurch gekennzeichnet, daß die Oxidationsfärbemittel unter den Oxidationsbasen und Gemischen von Oxidationsbasen und Kupplern ausgewählt sind.

12. Verfahren nach einem der vorhergehenden Ansprüche, dadurch gekennzeichnet, daß das Oxidationsmittel unter Wasserstoffperoxid, Harnstoffperoxid, Alkalimetallbromaten und Salzen von Persäuren ausgewählt ist.